# EUROPÄISCHE PATENTSCHRIFT

(11) **EP 3 579 903 B1**
(45) Veröffentlichungstag und Bekanntmachung des Hinweises auf die Patenterteilung: **21.12.2022**
(21) Anmeldenummer: 18701009.5
(22) Anmeldetag: 17.01.2018
(51) Int. Cl.: A61M 25/00, A61M 5/142, F04B 43/12, A61M 60/13, A61M 60/408, A61M 60/554, A61M 60/808, A61M 60/226

(54) **SPÜLSYSTEM**
FLUSHING SYSTEM
SYSTÈME DE RINÇAGE

(30) Priorität: 13.02.2017 DE 102017102829
(43) Veröffentlichungstag der Anmeldung: 18.12.2019
(73) Patentinhaber: Cardiobridge GmbH, 72379 Hechingen (DE)
(72) Erfinder: EPPLE, Klaus, 72414 Rangendingen (DE)
(74) Vertreter: DREISS Patentanwälte PartG mbB
(86) Internationale Anmeldenummer: PCT/EP2018/051053
(87) Internationale Veröffentlichungsnummer: WO 2018/145865

(56) Entgegenhaltungen:
- EP-A1- 0 459 113
- EP-A1- 0 711 570
- WO-A2-2009/055639
- US-A1- 2005 238 515

## Beschreibung

Die Erfindung betrifft ein Spülsystem für einen Katheter, insbesondere für einen Katheter einer Katheterpumpe, umfassend eine Zuführleitung mit einem Pumpabschnitt, eine Abführleitung mit einem Pumpabschnitt und eine Pumpe. Die Erfindung betrifft auch ein dazugehöriges Trägerelement, eine dazugehörige Konsole und ein Katheterpumpensystem.

Eine Katheterpumpe ist beispielsweise aus der EP 2 288 392 B1 bekannt. Die Katheterpumpe weist eine Antriebseinheit und einen Katheter auf. Der Katheter umfasst einen Pumpenkopf zum Einführen insbesondere in das arterielle Gefäßsystem wie zum Beispiel die Aorta oder das Herz auf, und eine verdrehbar angeordnete Rotorwelle zum Antreiben eines am Pumpenkopf vorgesehenen, expandierbaren Förderelements. Als rotierendes Förderelement kann ein Rotor mit ausklappbaren Propellern Verwendung finden, der am proximalen Ende des Katheters vorgesehen ist.

Katheterpumpen werden als temporäres Kreislaufunterstützungssystem in das arterielle Gefäßsystem wie zum Beispiel die Aorta von Patienten eingesetzt, insbesondere dann, wenn das natürliche Herz nicht in der Lage ist, den Körper mit ausreichend Sauerstoff versetztem Blut zu versorgen. Das Förderelement und die Rotorwelle werden dabei mit vergleichsweise hohen Drehzahlen im Bereich von 7000 bis 15000 Umdrehungen pro Minute und insbesondere im Bereich von 10000 bis 13000 Umdrehungen betrieben.

Um zu verhindern, dass sich Ablagerungen im Katheter bilden, um den Katheter und insbesondere die sich darin rotierende Rotorwelle zu schmieren, und um das Eindringen von Blut in den Katheter zu verhindern, wird der Katheter mit Spülflüssigkeit gespült. Hierzu wird ein Spülsystem mit dem Katheter verbunden. Ein solches Spülsystem ist beispielsweise aus der WO 2014/164136 A1 vorbekannt. Dabei werden eine Zuführleitung zum Zuführen von Spülfluid und eine Abführleitung zum Abführen von Spülfluid mit dem Katheter verbunden. Um eine Fluidförderung in der Zuführ- bzw. Abführleitung zu erzielen, ist jeweils eine Rotationsperistaltikpumpe für die Zuführleitung und die Abführleitung vorgesehen. Die beiden Pumpen werden hierbei separat angesteuert, um eine entsprechende Menge an Fluid in den Katheter zu fördern bzw. aus dem Katheter herauszuführen und um ein gewünschtes Verhältnis von zugeführtem zu abgeführtem Fluid zu erzielen. Dieses Spülsystem ist zum einen teuer und komplex im Aufbau, weil zwei Pumpen vorgesehen werden müssen, und zum anderen komplex zu steuern bzw. zu programmieren, weil beide Pumpen aufeinander abgestimmt werden müssen.

Aus der US 2005/0238515 A1 ist eine Peristaltikpumpe bekannt, die eine Zuführleitung und eine Abführleitung mit jeweils Pumpabschnitte aufweist, wobei die Pumpabschnitte einen unterschiedlichen Durchmesser aufweisen können.

Der vorliegenden Erfindung liegt deshalb die Aufgabe zugrunde, ein Spülsystem für einen Katheter bereitzustellen, welches den genannten Nachteilen des Standes der Technik Abhilfe verschafft.

Diese Aufgabe wird durch ein Spülsystem mit den Merkmalen des Anspruchs 1 gelöst. Es ist demnach vorgesehen, dass der Durchmesser des Pumpabschnitts der Abführleitung kleiner ist als der Durchmesser des Pumpabschnitts der Zuführleitung. Ferner ist vorgesehen, dass die Pumpe mit dem Pumpabschnitt der Zuführleitung zum Zuführen von Spülfluid zum Katheter zusammenwirkt. Schließlich ist vorgesehen, dass die Pumpe mit dem Pumpabschnitt der Abführleitung zum Abführen von Spülfluid aus dem Katheter zusammenwirkt.

Folglich ist nur eine Pumpe vorhanden, die sowohl zum Zuführen von Fluid durch die Zuführleitung in den Katheter hinein als auch zum Abführen von Fluid durch die Abführleitung aus dem Katheter hinaus ausgebildet ist. Dadurch, dass der Durchmesser des Pumpabschnitts der Abführleitung im Vergleich zum Durchmesser der Zuführleitung reduziert ist, kann das Verhältnis von zugeführtem Fluid zu abgeführtem Fluid eingestellt und angepasst werden, ohne dass eine zweite Pumpe vonnöten wäre. Der Pumpabschnitt der Abführleitung wirkt hierbei mit der Pumpe derart zusammen, dass eine Art Drosselstelle für das Spülfluid ausgebildet wird. Somit kann erreicht werden, dass ca. 2/3 des in den Katheter gepumpten Spülfluids in dem Körper verbleibt, in dem sich der Katheter befindet, und ca. 1/3 des Spülfluids durch den Katheter zurückfließt, und mittels der Abführleitung aus dem Katheter abgeführt wird.

Gemäß der Erfindung ist weiter vorgesehen, dass ein Trägerelement vorgesehen ist, an dem die Zuführleitung und die Abführleitung befestigt sind. Mittels des Trägerelements kann auf besonders einfache Art und Weise eine Wirkverbindung zwischen Zuführ- und Abführleitung und der Pumpe bereitgestellt werden, indem das Trägerelement in geeigneter Weise derart im Pumpenbereich angebracht wird, sodass die Pumpabschnitte der Zuführ- bzw. Abführleitung mit der Pumpe zusammenwirken können.

Dabei erstreckt sich das Trägerelement entlang einer Haupterstreckungsebene. In diesem Zusammenhang ist denkbar, dass sich die Zuführ- und Abführleitung in dieser Haupterstreckungsebene durch das Trägerelement erstrecken. Das Trägerelement ist folglich besonders kompakt aufgebaut und auf besonders einfache Art und Weise mit einer linearen Peristaltikpumpe verbindbar

Zudem ist vorgesehen, dass das Trägerelement einen Durchbruch aufweist, wobei sich die Pumpabschnitte der Zuführ- und Abführleitung entlang des Durchbruchs erstrecken und somit diesen überbrücken. Denkbar ist, dass die Finger der linearen Peristaltikpumpe in den Durchbruch eingreifen und somit mit den Pumpabschnitten, die den Durchbruch überbrücken, zusammenwirken.

Bei dem Katheter kann es sich einerseits, wie oben ausgeführt, um den Katheter einer Katheterpumpe handeln. Andererseits kann es sich auch um unterschiedlichste andere Katheter handeln, wie beispielsweise um einen Katheter eines Stent-Einführsystems.

Die Pumpe ist als lineare Peristaltikpumpe ausgebildet. Die Pumpe weist eine Nockenwelle mit einer Anzahl Nocken aufweist, wobei jeder Nocken mit jeweils einem Finger bewegungsgekoppelt ist. Zur Fluidförderung wirkt jeder Finger mit der Zuführleitung und der Abführleitung zusammen. Insbesondere drücken die Finger die Zuführleitung und die Abführleitung sequentiell zusammen, um eine Fluidströmung zu erzeugen. Mit der Bewegung der Finger wandert folglich auch der gequetschte Pumpabschnittsbereich, sodass eine Fluidströmung erzielt wird. Insgesamt kann folglich mittels nur einer Pumpe dem Katheter Fluid zugeführt bzw. Fluid aus dem Katheter abgeführt werden.

Vorteilhaft ist auch, wenn die Pumpabschnitte parallel zueinander verlaufen und in Betrieb von Spülfluid in entgegengesetzten Strömungsrichtungen durchströmt werden. Ein derartiges Spülsystem ist besonders einfach und kompakt aufgebaut.

Vorteilhafterweise weist die Zuführleitung bzw. die Abführleitung einen bogenförmigen Abschnitt auf, wobei der Abschnitt durch das Trägerelement verläuft, sodass die Zuführleitung bzw. Abführleitung an derselben Seite des Trägerelements in das Trägerelement hinein bzw. aus dem Trägerelement hinaus geführt ist. Die Zuführ- und/oder Abführleitung kann folglich an einer Trägerelementseite dem Trägerelement zugeführt werden und an derselben Seite aus dem Trägerelement hinaus geführt werden. Dies hat den Vorteil, dass beide Leitungen mittels nur einer Pumpe, und insbesondere mittels einer linearen Peristaltikpumpe, beaufschlagt werden können, so dass das Fluid in der Zuführleitung zur Zuführung gefördert und in der anderen Leitung zur Abführung gefördert wird. Denkbar ist allerdings auch, dass die Zuführ- und/oder Abführleitung an gegenüberliegenden Seite in das Trägerelement geführt werden bzw. aus diesem hinaus geführt werden.

Bevorzugt ist ferner, wenn eine die Pumpe aufweisende Konsole vorgesehen ist. Dabei können die Pumpabschnitte der Zuführ- und Abführleitung in eine Aufnahme in der Konsole derart einführbar sein, dass die Pumpe mit den Pumpabschnitten zur Fluidförderung zusammenwirken kann. Indem die Pumpabschnitte folglich in die Konsole eingelegt werden, kann eine Wirkverbindung zwischen Pumpe und Zuführsowie Abführleitung zur Fluidförderung in einen Katheter bzw. aus einem Katheter bereitgestellt werden.

Besonders bevorzugt ist dabei, wenn das Trägerelement samt Zuführ- und Abführleitung an der Konsole lösbar anlegbar ist. Die Zuführ- und Abführleitung sind im Gegensatz zur Konsole Verschleißartikel, da diese meist steril sein müssen. Für jeden Patient muss eine neue Zuführ- und Abführleitung verwendet werden. Insofern kann durch einfaches Entfernen bzw. Neuanordnen des Trägerelements samt Zuführ- und Abführleitung an der Konsole ein rascher Austausch von Zuführ- und Abführleitung vorgenommen werden.

Vorteilhafterweise weist die Konsole ein Schließelement zum Verschließen der Aufnahme auf, wobei die Pumpabschnitte erst dann mit der Pumpe zur Fluidförderung zusammenwirken, wenn das Schließelement seine Geschlossenstellung einnimmt. In diesem Zusammenhang ist denkbar, dass das Schließelement einen Wirkabschnitt aufweist, der den Fingern der linearen Peristaltikpumpe gegenüberliegend vorgesehen ist, sodass die Pumpabschnitte zwischen Wirkabschnitt und den Fingern vorgesehen sind.

Eine besonders bevorzugte Weiterbildung der Erfindung ergibt sich in diesem Zusammenhang daraus, dass das Schließelement ein Drückelement aufweist, das die Zuführleitung und/oder die Abführleitung unter Vorspannung gegen die Finger drückt. Dabei kann bzw. können ein oder mehrere Federelemente vorgesehen sein, um das Drückelement gegen die Zuführ- und oder Abführleitung zu beaufschlagen. Besonders denkbar ist, dass die Drückelemente gegenüber den Fingern einer linearen Peristaltikpumpe angeordnet sind, sodass die Drückelemente eine kontinuierliche Kraft auf die Pumpabschnitte in Richtung der Finger ausüben, während die Finger die Pumpabschnitte sequentiell mit einer Kraft in Richtung der Drückelemente beaufschlagen, um eine Fluidströmung zu erzeugen.

Die eingangs gestellte Aufgabe wird auch gelöst, durch ein Trägerelement mit den Merkmalen des Patentanspruchs 9. Dieser Verschleißartikel ist besonders einfach und austauschbar insbesondere an der Konsole einfach befestigbar.

Die eingangs gestellte Aufgabe wird ferner auch gelöst, durch eine Konsole mit den Merkmalen des Patentanspruchs 10. Die Pumpabschnitte der Zuführ- und Abführleitung sind dabei derart in die Aufnahme einführbar, dass nach dem Einführen eine Wirkverbindung zwischen der Pumpe und den Pumpabschnitten vorhanden ist, um eine Fluidströmung durch die Leitungen zu erzeugen.

Schließlich wird die eingangs gestellte Aufgabe auch durch ein Katheterpumpensystem mit den Merkmalen des Patentanspruchs 11 gelöst. Das Katheterpumpensystem umfasst hierbei eine Katheterpumpe mit einem Katheter und ein erfindungsgemäßes Spülsystem, wobei die Zuführleitung und die Abführleitung des Spülsystems mit dem Katheter fluidisch verbunden sind. Der Katheter der Katheterpumpe ist mittels des Spülsystems auf besonderes einfach und Art und Weise spülbar.

Weitere Einzelheiten und vorteilhafte Ausgestaltungen der Erfindung sind der folgenden Beschreibung zu entnehmen, anhand derer die in den Figuren dargestellte Ausführungsform der Erfindung näher beschrieben und erläutert ist. Es zeigen:
- Figur 1: eine Katheterpumpe mit einer Antriebseinheit und einem Katheter;
- Figur 2: eine Antriebseinheit einer Katheterpumpe in Betriebslage;
- Figur 3: eine Perspektivansicht einer Konsole eines erfindungsgemäßen Spülsystems mit einem daran befestigten Trägerelement und einem Schließelement in Geschlossenstellung;
- Figur 4: Ansicht gemäß Figur 3 mit dem Schließelement in Offenstellung, wobei das Trägerelement von der Konsole gelöst ist;
- Figur 5: eine perspektivische Ansicht des in den Figuren 3 und 4 gezeigten Trägerelements;
- Figur 6: eine weitere perspektivische Ansicht des in den Figuren 3 und 4 gezeigten Trägerelements;
- Figur 7: schematische perspektivische Ansicht einer Pumpe der in den Figuren 3 und 4 gezeigten Konsole; und
- Figur 8: schematische perspektivische Ansicht des Schließelements der in den Figuren 3 und 4 gezeigten Konsole.

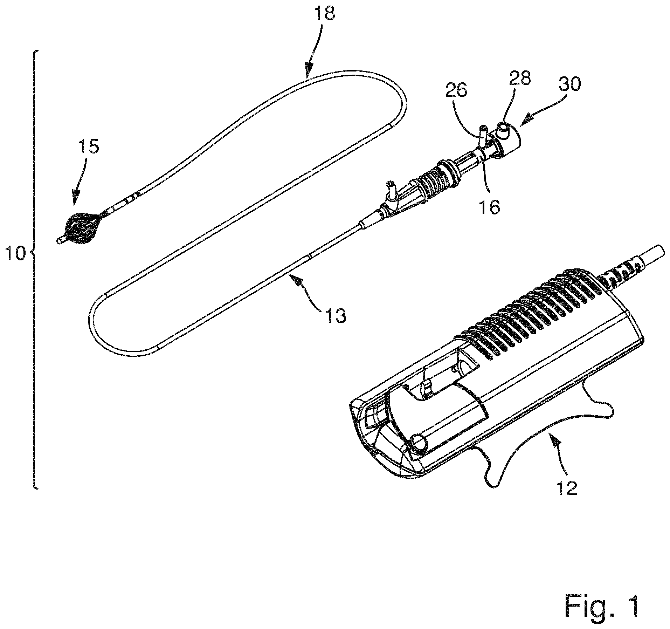

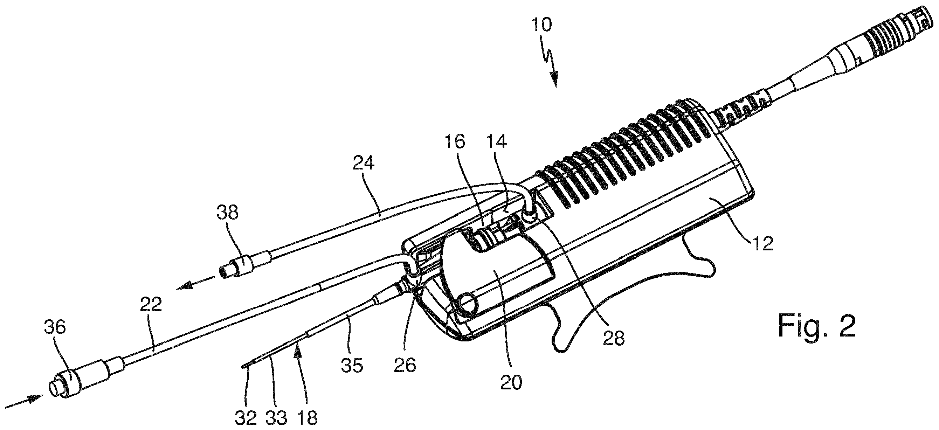

In den Figuren 1 und 2 ist eine Katheterpumpe 10 mit einer Antriebseinheit 12 und mit einem mit der Antriebseinheit 12 koppelbaren Katheter 18 gezeigt. Der Katheter 18 weist an seinem distalen Ende einen Pumpenkopf 15 zum Einführen in das arterielle Gefäßsystem wie zum Beispiel die Aorta auf. Der Katheter 18 ist dafür bestimmt, in den Körper eines Säugetiers, wie zum Beispiel eines Menschen, über die Oberschenkelarterie eingeführt zu werden und um zum Beispiel in der Aorta positioniert zu werden für eine Unterstützung des Kreislaufs des Herzens. Der Katheter 18 ist relativ lang, so dass er sich von der perkutanen Einführungsstelle in zum Beispiel der Oberschenkelarterie in die Leistengegend und bis hinauf zu dem Aortabogen erstrecken kann.

Im Katheter 18 ist eine Rotorwelle 32 vorgesehen, mittels der ein im Pumpenkopf 15 vorgesehenes Förderelement, beispielsweise einen Rotor mit ausklappbaren Propellern, in Rotation versetzbar ist. Die Antriebseinheit 12 weist eine Aufnahme 14 auf. Das proximale Ende 16 des Katheters 18 ist dabei in der Aufnahme 14 angeordnet und wird dort mittels eines Halteelements 20 sicher gehalten. An seinem proximalen Ende 16 sieht der Katheter 18 einen Kopplungsabschnitt 30 vor, der in die Antriebseinheit 12 einlegbar ist, mittels welcher letztlich die Rotorwelle 32, und damit das Förderelement, in Rotation versetzbar ist.

Ferner sind am distalen Ende 16 zwei Schläuche 22, 24 vorgesehen. Über den Schlauch 22 kann über einen Einlauf 26 Spülfluid in den Katheter 18 eingeleitet werden. Dieses Spülfluid wird durch den Katheter 18 zum Pumpenkopf 15 geleitet. Im Pumpenkopf 15 wird ein Teil dieses Spülfluids wieder durch den Katheter 18 zurückgeführt und über einen Auslauf 28 und den Schlauch 24 ausgelassen. Das rückgeführte Spülfluid wird dabei zwischen der sich im Betrieb drehenden Rotorwelle 32 und einem Innenkatheter 33 rückgeführt. Der Innenkatheter 33 wird dabei von einem Außenkatheter 35 umgeben, wobei Spülfluid über das Lumen zwischen dem Innenkatheter 33 und dem Außenkatheter 35 hin zum Pumpenkopf 15 gefördert wird.

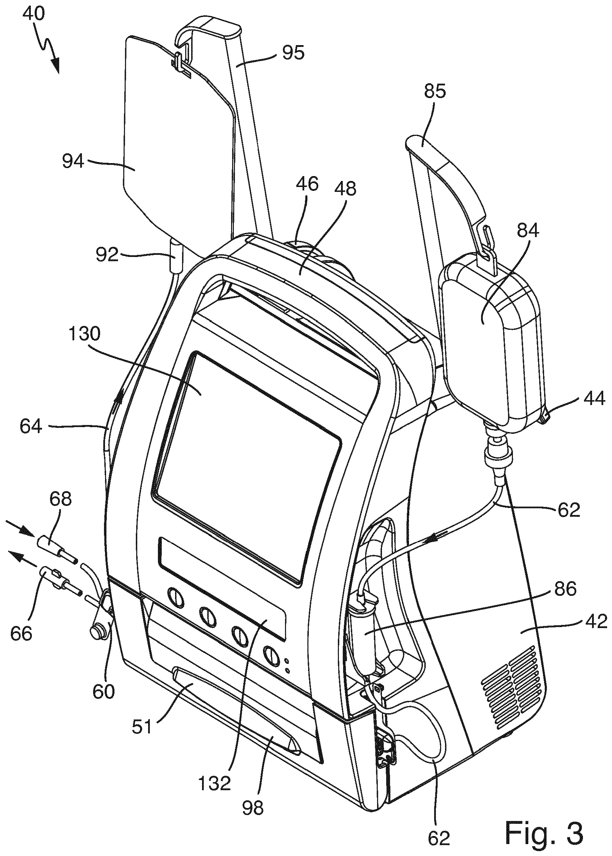

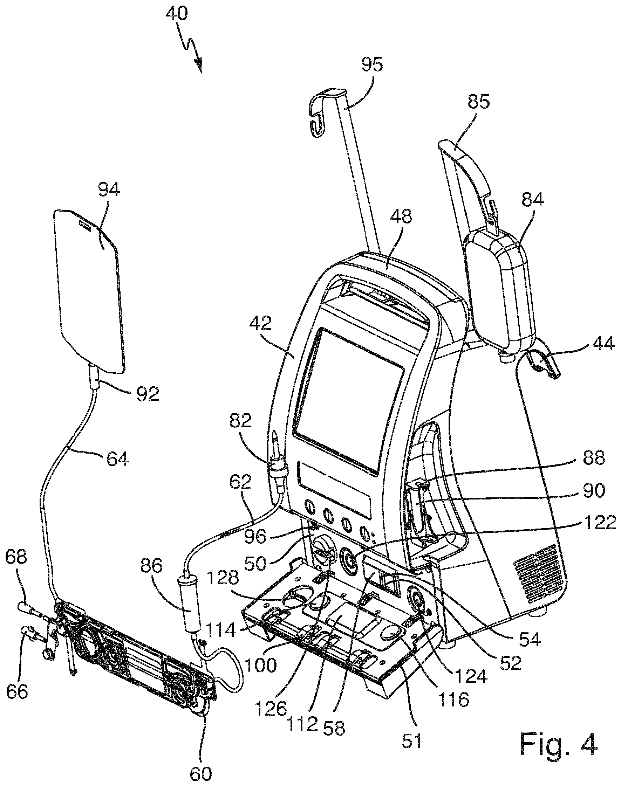

Die Schläuche 22, 24 weisen jeweils ein Anschlussstück 36, 38 auf. Diese Anschlussstücke 36, 38 sind mit einem Spülsystem verbindbar, welches im Folgenden näher beschrieben wird. Das Spülsystem ist in den Figuren 3 und 4 gezeigt und insgesamt mit dem Bezugszeichen 40 bezeichnet. Zunächst umfasst das Spülsystem 40 eine Konsole 42. Diese umfasst an ihrer Rückseite zwei Haken 44, 46 mittels welcher die Konsole beispielsweise an einem Krankhausbett befestigbar ist. Ferner umfasst die Konsole 42 einen Halteabschnitt 48, so dass die Konsole 42 von einer Bedienperson bequem getragen werden kann. Ferner umfasst die Konsole eine in Figur 4 zu erkennende Aufnahme 50, die von einem verschwenkbaren Schließelement 51 verschließbar ist.

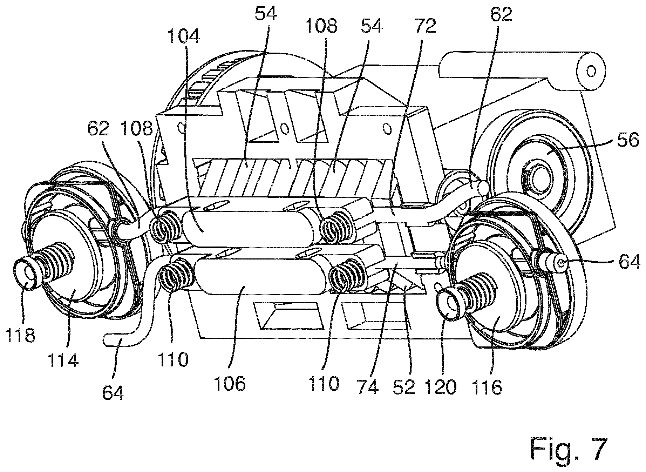

Weiterhin umfasst die Konsole 42 eine als lineare Peristaltikpumpe ausgebildete Pumpe 52. Diese Pumpe 52 wird im Folgenden anhand der Figuren 4 und 7 näher beschrieben: Zunächst umfasst die Pumpe 52 eine Nockenwelle 56, mit einer Anzahl nicht gezeigter Nocken. Die Nocken sind jeweils mit einem Finger 54 bewegungsgekoppelt. Diese Finger 54 bewegen sich in radialer Richtung, also senkrecht zur Drehachse der Nockenwelle 56. Die Finger 54 bewegen sich hierbei sequenziell, das heißt insgesamt bewegen sich die Finger 54 zusammen wellenförmig. In Figur 4 sind die Finger zu erkennen, die in die Aufnahme 50 ragen und von einer Bedeckung 58 bedeckt werden. In der Aufnahme 50 ist ein Trägerelement 60 mit einer Zuführleitung 62 und einer Abführleitung 64 anordenbar.

Das Trägerelement 60 sowie die Leitungen 62, 64 werden im Folgenden anhand der Figuren 3 bis 6 näher beschrieben. Zunächst umfasst die Zuführleitung 62 ein Anschlussstück 66, während die Abführleitung 64 ein Anschlussstück 68 umfasst. Das Anschlussstück 66 ist hierbei mit dem Anschlussstück 36 des Schlauchs 22 der Katheterpumpe 10 (vgl. Figur 2) verbindbar. Die Anschlussstücke 66 und 36 können einen Luer-Anschluss ausbilden. Ebenso ist das Anschlussstück 68 der Abführleitung 64 mit dem Anschlussstück 38 des Schlauchs 24 der Katheterpumpe 10 verbindbar. Die Anschlussstücke 38 und 68 können ebenfalls einen Luer-Anschluss ausbilden.

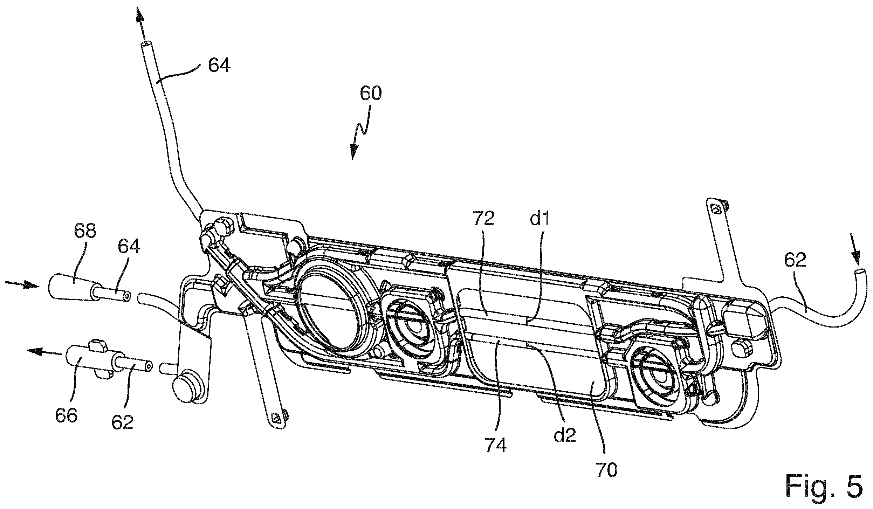

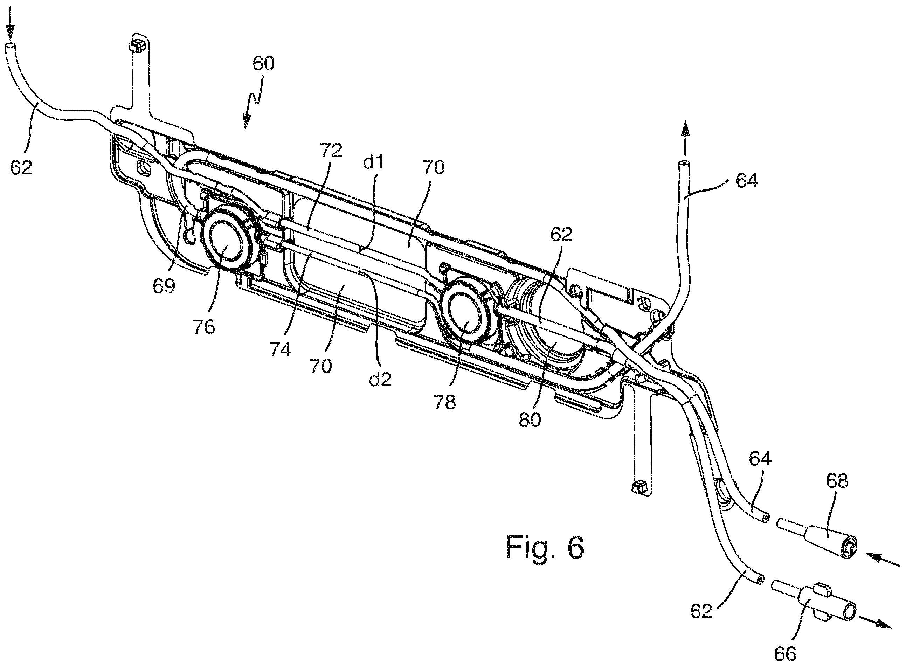

Wie den Figuren 5 und 6 deutlich zu entnehmen ist, erstreckt sich das Trägerelement 60 in einer Haupterstreckungsebene und ist kassettenartig aufgebaut. Das Trägerelement 60 besteht aus PET und wird durch Kunststoff Tiefziehen oder Spritzguss hergestellt. Die Zuführleitung 62 und die Abführleitung 64 sind am Trägerelement 60 befestigt bzw. verlaufen durch das Trägerelement 60 hindurch. Das Trägerelement 60 weist hierzu Kanäle auf, in denen die Leitungen 62, 64 geführt sind. Im mittleren Bereich weist das Trägerelement 60 einen rechteckförmigen Durchbruch 70 auf. Die Leitungen 62, 64 erstrecken sich entlang dieses Durchbruchs 70 und überbrücken diesen.

Im Bereich des Durchbruchs 70 umfasst die Zuführleitung 62 einen Pumpabschnitt 72. Gleichzeitig umfasst die Abführleitung 64 einen Pumpabschnitt 74. Der Pumpabschnitt 72 weist einen Durchmesser d1 auf, während der Pumpabschnitt 74 einen Durchmesser d2 aufweist. Der Durchmesser d1 ist hierbei größer als der Durchmesser d2. Die Funktion dieser Durchmesserdifferenz wird im Folgenden noch näher beschrieben werden.

Am Trägerelement 60 sind ferner zwei Membranen 76, 78, die insbesondere aus Silikon bestehen können, vorgesehen. Mittels dieser Membranen 76, 78 ist eine Druckmessung in der Zuführleitung 62 bzw. der Abführleitung 64 durchführbar, wie im Folgenden noch näher beschrieben werden wird. Ferner weist das Trägerelement 60 einen weiteren Durchbruch 80 auf. Dieser Durchbruch 80 wird nur von der Zuführleitung 62 überbrückt. Wie in den Figuren 3 und 4 ersichtlich ist, weist die Zuführleitung 62 ein weiteres Anschlussstück 82 auf. Mittels dieses Anschlussstücks 82 ist der Zuführschlauch 62 mit einem Beutel 84 verbindbar. Der Beutel 84 enthält Spülfluid. Der Beutel 84 ist an einem Befestigungshaken 85 der Konsole 42 befestigt. Ferner umfasst die Zuführleitung 62 eine Behälter 86 mit vergrößertem Durchmesser. Dieser Behälter 86 liegt zwischen dem Anschlussstück 82 und dem Trägerelement 60. Der Behälter 86 ist an einer Aufnahme 88 an der Konsole befestigbar und liegt an einem konsolenseitigen Fenster 90 an. Mittels dieser Anordnung kann eine Füllstandsmessung der Zuführleitung 62 beispielsweise mittels eines kapazitiven Sensors bereitgestellt werden. Die Abführleitung 64 weist ebenfalls ein Anschlussstück 92 auf. Das Anschlussstück 92 ist hierbei mit einem Auffangbeutel 94 für verbrauchtes Spülfluid verbindbar. Der Auffangbeutel 94 ist ebenfalls an einem Befestigungshaken 95 der Konsole 42 befestigt. Die Befestigungshaken 85, 95 samt den Beuteln 84, 94 sind an gegenüberliegenden Schmalseiten der Konsole 42 vorgesehen.

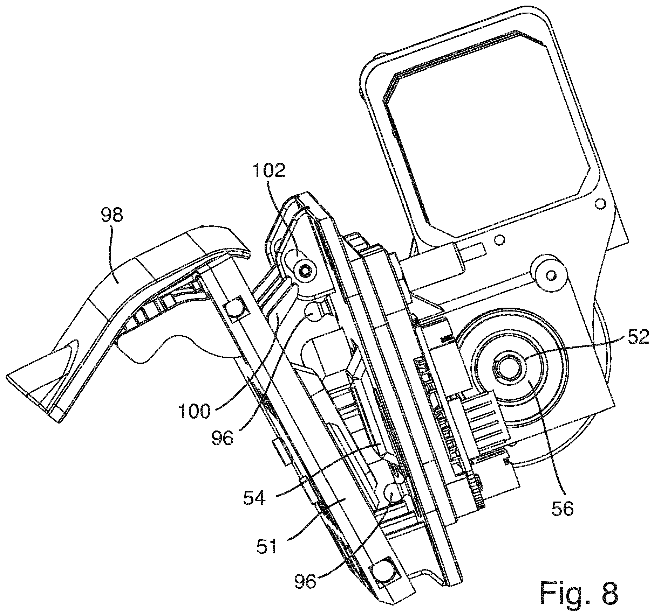

Zum Befestigen des Trägerelements 60 in der Aufnahme 50 weist die Konsole eine Anzahl Fixierungsbolzen 96 auf. Sobald das Trägerelement 60 in der Aufnahme 50 angeordnet ist, kann die Aufnahme 50 geschlossen werden, wie in Figur 3 gezeigt ist. Hierzu wird das Schließelement 51 aus der in Figur 4 gezeigten Offenstellung in seine in Figur 3 gezeigte Geschlossenstellung überführt. Zum Schließen des Schließelements 51 weist dieses eine Schließklappe 98 auf. Die Schließklappe 98 ist in Figur 8 besonders deutlich zu erkennen. Diese ist verschwenkbar. Um das Schließelement 51 zu schließen, wird die Schließklappe 98 verschwenkt, sodass Zähne 100 der Schließklappe 98 einen konsolenseitigen Befestigungssteg 102 hintergreifen, so dass das Schließelement 51 in der Geschlossenstellung sicher geschlossen ist.

Wie aus Figur 3 ersichtlich ist, wird die Zuführleitung 62 an einer Seite der Konsole 42 in dieser hinein geführt und an einer gegenüberliegenden Seite hinausgeführt. Demgegenüber wird die Abführleitung 64 an derselben Seite der Konsole 42 in diese hineingeführt bzw. aus dieser hinausgeführt, nämlich an der Seite, wo die Abführleitung 64 aus der Konsole 42 in Richtung der Katheterpumpe 10 hinausgeführt wird. Dementsprechend ist die Abführleitung 64 in einem Bogen (in Fig. 6 erkennbar) durch das Trägerelement 60 geführt, sodass die Abführleitung 64 einen bogenförmigen Abschnitt 69 aufweist.

Um die Zuführleitung 62 und die Abführleitung 64 gegen die Finger 54 der Pumpe 52 in der Geschlossenstellung des Schließelements 51 zu beaufschlagen, weist das Schließelement 51 jeweils ein Drückelement 104, 106 für die Zuführleitung 62 und die Abführleitung 64 auf. Diese Drückelemente 104, 106 sind in Figur 7 deutlich zu erkennen. Diese erstrecken sich dabei jeweils entlang der Pumpabschnitte 72, 74 und werden an den seitlichen Enden von jeweils mindestens einem Federelement 108, 110 gegen die Finger 54 beaufschlagt. Wie in Figur 4 ersichtlich ist, ist eine Bedeckung 112 über den Drückelementen 104, 106 angeordnet.

Ferner weist das Schließelement 51 jeweils ein ebenfalls in Figur 7 zu erkennendes Beaufschlagungselement 114, 116 auf, wobei auch hier jeweils eine Druckfeder 118, 120 vorgesehen ist. Die Beaufschlagungselemente 114, 116 wirken hierbei mit den Membranen 66, 68 sowie konsolenseitig vorgesehenen Sensorelementen 122, 124 (vgl. Figur 4) zusammen, so dass eine Druckmessung in der Zuführleitung 62 und in der Abführleitung 64 möglich ist. In die Aussparung 80 im Trägerelement 60 greifen in der Geschlossenstellung ein konsolenseitiges Sensorelement 126 sowie ein schließelementseitiger Steg 128 ein, die auf die Zuführleitung 62 so wirken, dass eine Luftblasenerkennung durchführbar ist. In der Zuführleitung 62 sollen nämlich möglichst keine Luftbläschen oder sonstige Gasbläschen durchgelassen werden. Eine Luftblasenerkennung kann hierbei beispielsweise mittels optischer Methoden oder mittels kapazitiver Messung durchgeführt werden.

Die Funktionsweise des Spülsystems 40 ist sodann wie folgt: Zunächst wird das Spülsystem 40 mit der Katheterpumpe 10 verbunden, um ein Katheterpumpensystem auszubilden. Dabei wird der Anschluss 66 der Zuführleitung 62 mit dem Anschluss 36 der Katheterpumpe verbunden. Gleichermaßen wird der Anschluss 68 der Abführleitung 64 mit dem Anschluss 38 der Katheterpumpe 10 verbunden. Sodann kann die Pumpe 52 gestartet werden. Daraufhin wird Spülfluid, ausgehend vom Beutel 84 über die Zuführleitung 62 den Schlauch 22 dem Katheter 18 der Katheterpumpe 10 zuführt. Die Spülfluid kann hierbei aus einer 20%igen sterilen Glukoselösung bestehen. Heparin kann dem Spülfluid hinzugefügt sein. Die Menge des Spülfluids kann auf 600-2500ml/24h im Betrieb, und beim Befüllen mit deutlich größerem Fluss, ca. 12000ml/24h eingestellt sein.

Der Katheter 10 wird sodann in distaler Richtung vom Spülfluid durchströmt. Ein Teil des Spülfluids wird daraufhin durch den Katheter 18 hindurch in proximaler Richtung zurückgeführt und wirkt dabei als Schmiermittel für die Rotorwelle 32 des Katheters 18. Ferner wird das Eindringen von Blut in den Katheter 18 durch das Spülfluid wirksam verhindert. Über den Schlauch 24 und die Rückführleitung 64 wird das verbrauchte Spülfluid sodann dem Auffangbeutel 94 zugeführt. Dadurch, dass der Durchmesser d2 des Pumpenabschnitts 74 der Rückführleitung 64 kleiner ist als der Durchmesser d1 des Pumpenabschnitts 72 der Zuführleitung 62, wirkt der Pumpenabschnitt 64 mit der Pumpe 52 als Drosselstelle zusammen. Somit wird ca. ein Drittel des in den Katheter 18 gepumpten Spülfluids wieder aus dem Katheter herausgepumpt, während zwei Drittel des Spülfluids in den Kreislauf des Patienten eindringend, wobei zugleich verhindert wird, dass Blut in den Katheter 18 eindringt. Dementsprechend ist die Querschnittsfläche des Durchmessers d2 des Pumpenabschnitts 74 der Rückführleitung 64 auch ungefähr nur ein Drittel des der Querschnittsfläche mit dem Durchmesser d1 des Pumpenabschnitts 72 der Zuführleitung 62.

Jeder Finger 54 der Pumpe 52 wirkt dabei auf beide Pumpenabschnitte 72 und 74 gleichermaßen. Die lineare Peristaltikpumpe 52 ist eine Verdrängerpumpe, bei der das zu fördernde Spülfluid durch äußere mechanische Verformung der Leitungen 62, 64 bzw. deren Pumpabschnitte 72, 74 hindurchgedrückt wird, wobei der zusammengedrückte Abschnitt der Pumpabschnitte 72, 74 "wandert", aufgrund der sequenziellen axialen Bewegung der Finger 54. Mittels der Sensorelemente 122, 124 ist eine Druckmessung in den Leitungen 62, 64 möglich, während mittels des Sensorelements 126 eine Luftblasenerkennung in der Zuführleitung 62 möglich ist.

Die hauptsächlichen Funktionen der Konsole 42 sind, die Drehzahl der Katheterpumpe 10 bzw. die Durchflussmenge des Spülfluids zu überwachen und zu steuern. Die Konsole 42 weist an einer Breitseite zwei Monitore auf. Der erste Monitor 130 dient als Hauptmonitor und ist als Touchscreen ausgebildet. Alle Steuerungs- und Überwachungsparameter für das System werden am Monitor 130 angezeigt. Ein Betriebssystem, wie beispielsweise Windows, dient hierbei als Eingabesystem auf dem Bildschirm. Ein davon getrenntes Betriebssystem, welches speziell für die Medizinelektronik ausgebildet ist, dient zur Steuerung der Katheterpumpe 10 und/oder des Spülsystems 40. Die Steuerung für die Medizinelektronik dient hierbei insbesondere zur Drehzahlkontrolle der Katheterpumpe 10 bzw. der Pumpe 52 des Spülsystems 40. Sollte der Monitor 130 aus irgendwelchen Gründen ausfallen, weist die Konsole 42 einen zweiten Notmonitor 132 auf. Dieser hat nur wenige Funktionen, wie beispielsweise eine Anzeige der Drehzahl der Katheterpumpe 10. Mittels dieses sogenannten SUI (Sicherheits-User-Interface) kann der Betrieb der Katheterpumpe 10 und/oder des Spülsystems 40 lediglich beendet werden, nicht jedoch gestartet werden.

## Patentansprüche

1. Spülsystem (40) für einen Katheter (18), umfassend eine Zuführleitung (62) mit einem Pumpabschnitt, eine Abführleitung (64) mit einem Pumpabschnitt und eine Pumpe (52),
**dadurch gekennzeichnet,**
**dass** der Durchmesser d2 des Pumpabschnitts (74) der Abführleitung (64) kleiner ist als der Durchmesser d1 des Pumpabschnitts (72) der Zuführleitung (62),
**dass** die Pumpe (52) mit dem Pumpabschnitt (72) der Zuführleitung (62) zum Zuführen von Spülfluid zum Katheter (18) zusammenwirkt,
**dass** die Pumpe (52) mit dem Pumpabschnitt (74) der Abführleitung (64) zum Abführen von Spülfluid aus dem Katheter (18) zusammenwirkt,
wobei ein Trägerelement (60) vorgesehen ist, an dem die Zuführleitung (62) und die Abführleitung (64) befestigt sind,
wobei sich das Trägerelement (60) entlang einer Haupterstreckungsebene erstreckt, wobei die Zuführleitung (62) und Abführleitung (64) in dieser Haupterstreckungsebene durch das Trägerelement (60) verlaufen,
wobei das Trägerelement (60) einen Durchbruch (70) aufweist, und wobei sich die Pumpabschnitte (72, 74) der Zuführleitung (62) und der Abführleitung (64) entlang des Durchbruchs (70) erstrecken, wobei die Pumpe (52) als lineare Peristaltikpumpe ausgebildet ist, wobei die Pumpe (52) eine Nockenwelle (56) mit einer Anzahl Nocken aufweist, wobei jeder Nocken mit jeweils einem Finger (54) bewegungsgekoppelt ist, wobei jeder Finger (54) zur Fluidförderung mit der Zuführleitung (62) und der Abführleitung (64) zusammenwirkt, und wobei die Finger (54) die Zuführleitung (62) und die Abführleitung (64) sequentiell zusammendrücken, um eine Fluidströmung zu erzeugen.

2. Spülsystem (40) nach einem der vorhergehenden Ansprüche, wobei die Pumpabschnitte (72, 74) parallel zueinander verlaufen und in Betrieb von Spülfluid in entgegengesetzten Strömungsrichtungen durchströmt werden.

3. Spülsystem (40) nach einem der vorhergehenden Ansprüche, wobei die Zuführleitung (62) bzw. die Abführleitung (64) einen bogenförmigen Abschnitt (69) aufweist, wobei der Abschnitt durch das Trägerelement (60) verläuft, sodass die Zuführleitung (62) bzw. Abführleitung (64) derselben Seite in das Trägerelement (60) hinein bzw. aus dem Trägerelement (60) hinaus geführt ist.

4. Spülsystem (40) nach einem der vorhergehenden Ansprüche, wobei eine die Pumpe (52) aufweisende Konsole (42) vorgesehen ist, wobei die Pumpabschnitte (72, 74) in eine Aufnahme (50) in der Konsole (42) derart einführbar sind, dass die Pumpe (52) mit den Pumpabschnitten (72, 74) zur Fluidförderung zusammenwirken kann.

5. Spülsystem (40) nach Anspruch 4, wobei das Trägerelement (60) samt Zuführleitung (62) und Abführleitung (64) an der Konsole (42) lösbar anlegbar ist.

6. Spülsystem (40) nach Anspruch 4 oder 5, wobei die Konsole (42) ein Schließelement (51) zum Verschließen der Aufnahme (50) aufweist, wobei die Pumpabschnitte (72, 74) erst dann mit der Pumpe (52) zur Fluidförderung zusammenwirken, wenn das Schließelement (51) seine Geschlossenstellung einnimmt.

7. Spülsystem (40) nach Anspruch 6, wobei das Schließelement (51) ein Drückelement (104, 106) aufweist, das die Zuführleitung (62) und/oder die Abführleitung (64) unter Vorspannung gegen die Finger (54) drücken.

8. Spülsystem (40) nach Anspruch 7, wobei jeweils ein Drückelement (104, 106) für die Zuführleitung (62) und die Abführleitung (64) vorgesehen ist.

9. Trägerelement (60) zum Einführen in die Aufnahme (50) der die Pumpe (52) aufweisenden Konsole (42) eines Spülsystems (40) nach einem der Ansprüche 4 bis 8, wobei am Trägerelement (60) die Zuführleitung (62) und die Abführleitung (64) befestigt sind,
wobei sich das Trägerelement (60) entlang einer Haupterstreckungsebene erstreckt,
wobei die Zuführleitung (62) und Abführleitung (64) in dieser Haupterstreckungsebene durch das Trägerelement (60) verlaufen,
wobei das Trägerelement (60) einen Durchbruch (70) aufweist, und
wobei sich die Pumpabschnitte (72, 74) der Zuführleitung (62) und der Abführleitung (64) entlang des Durchbruchs (70) erstrecken und den Durchbruch (70) derart überbrücken, dass bei in die Konsole (42) eingesetztem Trägerelement (60) die Finger (54) der Pumpe (52) mit den den Durchbruch (70) überbrückenden Pumpabschnitten (72, 74) zusammenwirken.

10. Konsole (42) für ein Spülsystem (40) nach einem der Ansprüche 4 bis 8, wobei die Konsole (42) die Pumpe (52) und die Aufnahme (50) für das Trägerelement (60) nach Anspruch 9 aufweist,
wobei die Pumpe (52) als lineare Peristaltikpumpe ausgebildet ist, die eine Nockenwelle (56) mit einer Anzahl Nocken aufweist, wobei jeder Nocken mit jeweils einem Finger (54) bewegungsgekoppelt ist, und
wobei die Pumpe (52) und deren Finger (54) derart ausgebildet sind, dass die Finger (54) bei in der Aufnahme eingeführtem Trägerelement (60) mit den den Durchbruch (70) des Trägerelements (60) überbrückenden Pumpabschnitten (72, 74) zusammenwirken und die Zuführleitung (62) und die Abführleitung (64) sequentiell zusammendrücken, um eine Fluidströmung zu erzeugen.

11. Katheterpumpensystem umfassend eine Katheterpumpe (10) mit einem Katheter (18) und ein Spülsystem (40) nach einem der Ansprüche 1 bis 8, wobei die Zuführleitung (62) und die Abführleitung (64) mit dem Katheter (18) fluidisch verbunden sind.

## Claims

1. A flushing system (40) for a catheter (18), comprising a supply line (62) having a pump portion, a discharge line (64) having a pump portion, and a pump (52),
**characterized in that**
the diameter d2 of the pump portion (74) of the discharge line (64) is smaller than the diameter d1 of the pump portion (72) of the supply line (62),
the pump (52) cooperates with the pump portion (72) of the supply line (62) in order to supply flushing fluid to the catheter (18),
the pump (52) cooperates with the pump portion (74) of the discharge line (64) in order to discharge flushing fluid from the catheter (18),
a support element (60) to which the supply line (62) and the discharge line (64) are fastened being provided,
the support element (60) extending along a main plane of extension, the supply line (62) and the discharge line (64) extending through the support element (60) in this main plane of extension,
the support element (60) having an opening (70), and the pump portions (72, 74) of the supply line (62) and discharge line (64) extending along the opening (70), the pump (52) being designed as a linear peristaltic pump, the pump (52) comprising a camshaft (56) having a number of cams, each cam being motion-coupled to one finger (54) in each case, each finger (54) cooperating with the supply line (62) and discharge line (64) in order to convey fluid, and the fingers (54) sequentially compressing the supply line (62) and discharge line (64) in order to generate a fluid flow.

2. The flushing system (40) according to any of the preceding claims, wherein the pump portions (72, 74) extend in parallel with one another and flushing fluid flows through said portions during operation in opposite directions of flow.

3. The flushing system (40) according to any of the preceding claims, wherein the supply line (62) or the discharge line (64) has an arc-shaped portion (69), the portion extending through the support element (60) such that the supply line (62) or discharge line (64) of the same side is guided into the support element (60) or out of the support element (60), respectively.

4. The flushing system (40) according to any of the preceding claims, wherein a console (42) comprising the pump (52) is provided, wherein the pump portions (72, 74) can be inserted into a receptacle (50) in the console (42) such that the pump (52) can cooperate with the pump portions (72, 74) in order to convey fluid.

5. The flushing system (40) according to claim 4, wherein the support element (60) together with the supply line (62) and discharge line (64) can be releasably attached to the console (42).

6. The flushing system (40) according to claim 4 or claim 5, wherein the console (42) comprises a closing element (51) for closing the receptacle (50), wherein the pump portions (72, 74) only cooperate with the pump (52) in order to convey fluid when the closing element (51) assumes its closed position.

7. The flushing system (40) according to claim 6, wherein the closing element (51) comprises a pressing element (104, 106) that presses the supply line (62) and/or the discharge line (64) against the fingers (54) under preload.

8. The flushing system (40) according to claim 7, wherein a pressing element (104, 106) is provided for the supply line (62) and for the discharge line (64).

9. A support element (60) for insertion into the receptacle (50) of the console (42), comprising the pump (52), of a flushing system (40) according to any of claims 4 to 8, wherein the supply line (62) and the discharge line (64) are fastened to the support element (60),
wherein the support element (60) extends along a main plane of extension,
wherein the supply line (62) and the discharge line (64) extend through the support element (60) in said main plane of extension,
wherein the support element (60) has an opening (70), and
wherein the pump portions (72, 74) of the supply line (62) and discharge line (64) extend along the opening (70) and bridge the opening (70) in such a way that, when the support element (60) is inserted into the console (42), the fingers (54) of the pump (52) cooperate with the pump portions (72, 74) bridging the opening (70).

10. A console (42) for a flushing system (40) according to any of claims 4 to 8, wherein the console (42) comprises the pump (52) and the receptacle (50) for the support element (60) according to claim 9,
wherein the pump (52) is designed as a linear peristaltic pump comprising a camshaft (56) having a number of cams, each cam being motion-coupled to one finger (54) in each case, and
wherein the pump (52) and the fingers (54) thereof are designed such that the fingers (54), when the support element (60) is inserted in the receptacle, cooperate with the pump portions (72, 74) bridging the opening (70) of the support element (60) and sequentially compress the supply line (62) and discharge line (64) in order to generate a fluid flow.

11. A catheter pump system comprising a catheter pump (10) having a catheter (18) and a flushing system (40) according to any of claims 1 to 8, wherein the supply line (62) and the discharge line (64) are fluidically connected to the catheter (18).

## Revendications

1. Système de rinçage (40) pour un cathéter (18), comprenant une conduite d'amenée (62) avec une partie à pompe, une conduite d'évacuation (64) avec une partie à pompe et une pompe (52),
**caractérisé en ce**
**que** le diamètre d2 de la partie à pompe (74) de la conduite d'évacuation (64) est inférieur au diamètre d1 de la partie à pompe (72) de la conduite d'amenée (62),
**que** la pompe (52) coopère avec la partie à pompe (72) de la conduite d'amenée (62) pour amener un fluide de rinçage au cathéter (18),
**que** la pompe (52) coopère avec la partie à pompe (74) de la conduite d'évacuation (64) pour évacuer le fluide de rinçage du cathéter (18),
dans lequel un élément de support (60) est prévu, sur lequel la conduite d'amenée (62) et la conduite d'évacuation (64) sont fixées,
dans lequel l'élément de support (60) s'étend le long d'un plan d'étendue principal, dans lequel les conduite d'amenée (62) et conduite d'évacuation (64) s'étendent dans ce plan d'étendue principal à travers l'élément de support (60),
dans lequel l'élément de support (60) présente un passage (70), et dans lequel les parties à pompe (72, 74) de la conduite d'amenée (62) et de la conduite d'évacuation (64) s'étendent le long du passage (70), dans lequel la pompe (52) est réalisée sous la forme d'une pompe péristaltique linéaire, dans lequel la pompe (52) présente un arbre à cames (56) avec un certain nombre de cames, dans lequel chaque came est accouplée en mouvement à respectivement un doigt (54),
dans lequel chaque doigt (54) coopère avec la conduite d'amenée (62) et la conduite d'évacuation (64) pour le transport de fluide, et dans lequel les doigts (54) compriment la conduite d'amenée (62) et la conduite d'évacuation (64) de manière séquentielle, afin de produire un écoulement de fluide.

2. Système de rinçage (40) selon l'une quelconque des revendications précédentes, dans lequel les parties à pompe (72, 74) s'étendent parallèlement l'une à l'autre et lors d'un fonctionnement sont traversées par le fluide de rinçage dans des directions d'écoulement opposées.

3. Système de rinçage (40) selon l'une quelconque des revendications précédentes, dans lequel la conduite d'amenée (62) ou la conduite d'évacuation (64) présente une partie arquée (69), dans lequel la partie s'étend à travers l'élément de support (60), de sorte que la conduite d'amenée (62) ou conduite d'évacuation (64) est guidée à l'intérieur de l'élément de support (60) ou à l'extérieur de l'élément de support (60) du même côté.

4. Système de rinçage (40) selon l'une quelconque des revendications précédentes, dans lequel une console (42) présentant la pompe (52) est prévue, dans lequel les parties à pompe (72, 74) peuvent être introduites dans un logement (50) dans la console (42) de telle sorte que la pompe (52) peut coopérer avec les parties à pompe (72, 74) pour le transport de fluide.

5. Système de rinçage (40) selon la revendication 4, dans lequel l'élément de support (60) accompagné de la conduite d'amenée (62) et de la conduite d'évacuation (64) peut être placé de manière détachable sur la console (42).

6. Système de rinçage (40) selon la revendication 4 ou 5, dans lequel la console (42) présente un élément de fermeture (51) pour la fermeture du logement (50), dans lequel les parties à pompe (72, 74) ne coopèrent avec la pompe (52) pour le transport de fluide que lorsque l'élément de fermeture (51) occupe sa position fermée.

7. Système de rinçage (40) selon la revendication 6, dans lequel l'élément de fermeture (51) présente un élément de pression (104, 106), qui pressent la conduite d'amenée (62) et/ou la conduite d'évacuation (64) sous précontrainte contre les doigts (54).

8. Système de rinçage (40) selon la revendication 7, dans lequel respectivement un élément de pression (104, 106) est prévu pour la conduite d'amenée (62) et la conduite d'évacuation (64).

9. Elément de support (60) à introduire dans le logement (50) de la console (42) présentant la pompe (52) d'un système de rinçage (40) selon l'une quelconque des revendications 4 à 8,
dans lequel la conduite d'amenée (62) et la conduite d'évacuation (64) sont fixées sur l'élément de support (60),
dans lequel l'élément de support (60) s'étend le long d'un plan d'étendue principal,
dans lequel les conduite d'amenée (62) et conduite d'évacuation (64) s'étendent dans ce plan d'étendue principal à travers l'élément de support (60),
dans lequel l'élément de support (60) présente un passage (70), et
dans lequel les parties à pompe (72, 74) de la conduite d'amenée (62) et de la conduite d'évacuation (64) s'étendent le long du passage (70) et comblent le passage (70) de telle sorte que lorsque l'élément de support (60) est inséré dans la console (42), les doigts (54) de la pompe (52) coopèrent avec les parties à pompe (72, 74) comblant le passage (70).

10. Console (42) pour un système de rinçage (40) selon l'une quelconque des revendications 4 à 8, dans laquelle la console (42) présente la pompe (52) et le logement (50) pour l'élément de support (60) selon la revendication 9,
dans laquelle la pompe (52) est réalisée sous la forme d'une pompe péristaltique linéaire, qui présente un arbre à cames (56) avec un certain nombre de cames, dans laquelle chaque came est accouplée en mouvement à respectivement un doigt (54), et
dans laquelle la pompe (52) et les doigts (54) de celle-ci sont réalisés de telle sorte que les doigts (54) lorsque l'élément de support (60) est introduit dans le logement coopèrent avec les parties à pompe (72, 74) comblant le passage (70) de l'élément de support (60) et compriment la conduite d'amenée (62) et la conduite d'évacuation (64) de manière séquentielle, afin de produire un écoulement de fluide.

11. Système de pompe à cathéter comprenant une pompe à cathéter (10) avec un cathéter (18) et un système de rinçage (40) selon l'une quelconque des revendications 1 à 8, dans lequel la conduite d'amenée (62) et la conduite d'évacuation (64) sont reliées fluidiquement au cathéter (18).
